# EUROPÄISCHE PATENTSCHRIFT

(11) **EP 1 977 859 B1**
(45) Veröffentlichungstag und Bekanntmachung des Hinweises auf die Patenterteilung: **12.08.2009**
(21) Anmeldenummer: 07105772.3
(22) Anmeldetag: 05.04.2007
(51) Int. Cl.: B24C 1/00

(54) **Vorrichtung und Verfahren zur Oberflächenbearbeitung bzw. Oberflächenbehandlung mittels Trockeneisgranulat**
Device and method for processing surfaces or surface processing using dry ice granules
Dispositif et procédé destinés au traitement de surface ou à la manipulation de la surface au moyen d'un granulé de glace sèche

(43) Veröffentlichungstag der Anmeldung: 08.10.2008
(60) Teilanmeldung: 08101733.7
(73) Patentinhaber: Rotstein, Rosa, 80797 München (DE)
(72) Erfinder: Rotstein, Rosa, 80797 München (DE)
(74) Vertreter: Farago, Peter Andreas

(56) Entgegenhaltungen:
- EP-A- 0 234 365
- WO-A-90/14927
- WO-A-20/04037098
- US-A1- 2002 068 511
- US-A1- 2006 178 092
- US-B1- 6 174 225

## Beschreibung

Die vorliegende Erfindung betrifft ein Verfahren zur. Oberflächenbehandlung mittels Trockeneisgranulat.

### Stand der Technik

Trockeneis bzw. Trockeneisgranulat besteht, wie dem Fachmann wohl bekannt, aus einem Kohlendioxid (handelsüblich auch Kohlensäure genannt) in festem Zustand, das üblicherweise in Form von Pellets in einer Größe von etwa 3 mm erhältlich ist.

Aus der DE 10 2004 045 770 B3 ist, wie in Fig. 1 schematisch dargestellt, eine Vorrichtung zum bestrahlen einer Fläche mit einem Gemisch aus Treibgas (Transportluft) und Trockeneisgranulat bekannt, die nach dem Druckstrahlprinzip bzw. Einschlauchprinzip arbeitet.

Die bekannte Vorrichtung umfasst einen Einlass 6 für die Treibgasversorgung, der über einen durch eine Rotationskammer 90 verlaufenden Strömungsweg mit einem Auslass 22 in Verbindung steht, an den ein Transportschlauch 8 zum Ausgeben des Gemisches aus Treibgas und Trockeneisgranulat über eine Strahlpistole 9 mit einer Lavaldüse 10 anschließbar ist.

Darüber hinaus umfasst die bekannte Vorrichtung eine Dosiereinrichtung 20 zum Einbringen des Trockeneisgranulats in den Strömungsweg des Treibgases, wobei die Dosiereinrichtung 20 eine motorisch um eine Mittelachse drehbare Dosierscheibe 30 umfasst, die zwischen einem plattenförmigen Einströmteil 32 und einem plattenförmigen Ausströmteil 34 angeordnet ist und eine Vielzahl von Aufnahmekammern 44 aufweist, die in einer ersten Drehstellung der Dosierscheibe 30 fluchtend zu einer Zufuhrkammer 57 des Ausströmteiles 34 und in einer zweiten Drehstellung der Dosierscheibe 30 zwischen einer Einströmkammer 71 des Einströmteiles 32 und einer Ausströmkammer 59 des Ausströmteiles 34 um eine Antriebsachse 41 positionierbar sind. Zur Drehung der Dosierscheibe 30 wird ein herkömmlicher Motor 36 mit einer Drehzahlregelung 39 vorgesehen.

Die Zufuhrkammer 57 ist mit einem trichterförmigen Vorratsbehälter 1 zum Einbringen von Trockeneisgranulat in die Zufuhrkammer 57 verbunden, wobei der Vorratsbehälter mit herkömmlichen Trockeneis-Pellets 2 ausgefüllt ist, die unter Einwirkung der Schwerkraft und mit Unterstützung eines Rüttlers 86 in die Zuführkammer 57 gelangen können. Von der Zuführkammer 57 gelangen die Pellets 2 unter Einwirkung der Schwerkraft in eine der Aufnahmekammern 44 in der ersten Drehstellung und werden sodann durch die Drehung der Dosierscheibe 30 in die zweite Drehstellung befördert, so dass sie in die Durchströmungsrichtung 93 in Richtung des Auslasses 22 und zur Strahlpistole 9 hin befördert werden, um anschließend druckbeaufschlagt auf die zu reinigende bzw. zu behandelnde Fläche 28 aufzutreffen.

Die Vorrichtung der DE 10 2004 045 770 B3 ist jedoch insofern nachteilig, da ihre Reinigungsleistung relativ begrenzt ist und der Trockeneisverbrauch relativ hoch ist. Eine Erhöhung der Reinigungsleistung der Vorrichtung gemäß DE 10 2004 045 770 B3 wäre zwar durch die Steigerung der Menge an Treibgas möglich, was allerdings eine entsprechende Ausgestaltung und Dimensionierung der Druckluftquelle mit nachteiligen Auswirkungen für die Handhabbarkeit der Vorrichtung erfordern würde. Darüber hinaus ist die Vorrichtung gemäß der DE 10 2004 045 770 B3 nicht für topische und schonende Anwendungen zur kosmetischen Behandlung bzw. Reinigung der Haut nicht geeignet.

Dokument US 2006-178092 beschreibt ein Verfahren zur Oberflächenbearbeitung bzw. Oberflächenbehandlung mittels Trockeneisgranulat mit einer Vorrichtung umfassend: einen Vorratsbehälter zur Aufnahme von Trockeneis-Pellets, einen Einlasse für die Zuführung von Druckluft, einen Auslass zur Zuleitung eines Gemisches aus Druckluft und Trockeneisgranulat an eine Strahlpistole und eine dazwischenliegende Ausströmkammer, wobei die Trockeneis-Pellets vor der Zuführung in die Ausströmkammer (59) zu einer Größe in einem vorbestimmten Bereich zerkleinert werden, wobei die Größe der zerkleinerten Trockeneis-Pellets in einem Bereich von etwa 1 mm bis etwa 100 µ liegt.

Der Erfindung liegt daher die Aufgabe zugrunde, eine Vorrichtung und ein Verfahren zur Oberflächenbearbeitung bzw. Oberflächenbehandlung mittels Tockeneisgranulat zu schaffen, die bzw. das die oben angeführten Probleme des Standes der Technik vermeidet.

Im Rahmen dieser Aufgabe besteht eine besondere Aufgabe der vorliegenden Erfindung darin, ein Verfahren

für topische und schonende Anwendungen der kosmetischen Behandlung bzw. Reinigung der Haut zu schaffen.

Die obigen Aufgaben und weitere der nachfolgenden Beschreibung zu entnehmende Aufgaben werden durch die Verwendung eines Verfahrens nach Anspruch 1 gelöst. Vorteilhafte Weiterbildungen der gegenwärtigen Erfindung sind Gegenstand der Unteransprüche.

### Kurzbeschreibung der Figuren

Weitere Merkmale und Vorteile der vorliegenden Erfindung sowie die Wirkungsweise der exemplarischen Ausführungsform der vorliegenden Erfindung werden unten mit Bezug auf die begleitenden Zeichnungen beschrieben. Die begleitenden Zeichnungen veranschaulichen die vorliegende Erfindung und dienen zusammen mit der Beschreibung weiterhin dazu, die Grundsätze der Erfindung zu erklären und es einem Fachmann auf dem betreffenden Gebiet zu ermöglichen, die Erfindung zu verwenden.

Dabei zeigen:
Fig. 1 eine schematische Darstellung einer bekannten Vorrichtung gemäß der DE 10 2004 045 770 B3;
Fig. 2 eine schematische Darstellung einer Vorrichtung zur Oberflächenbearbeitung bzw. Oberflächenbehandlung mittels Trockeneisgranulat; und
Fig. 3 eine schematische Darstellung einer weiteren Vorrichtung zur Oberflächenbearbeitung bzw. Oberflächenbehandlung mittels Trockeneisgranulat.

### Beschreibung der bevorzugten Ausführungsformen der Erfindung

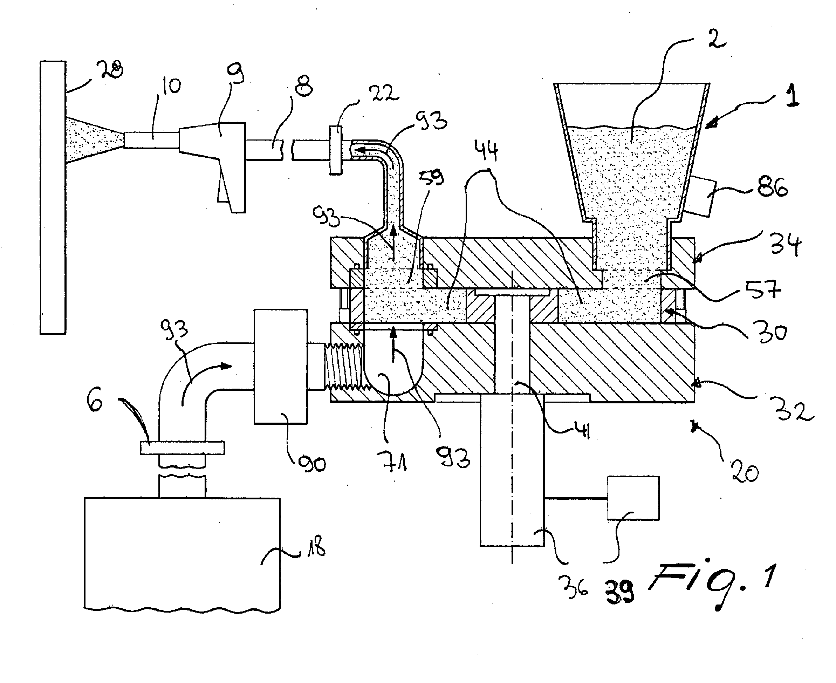

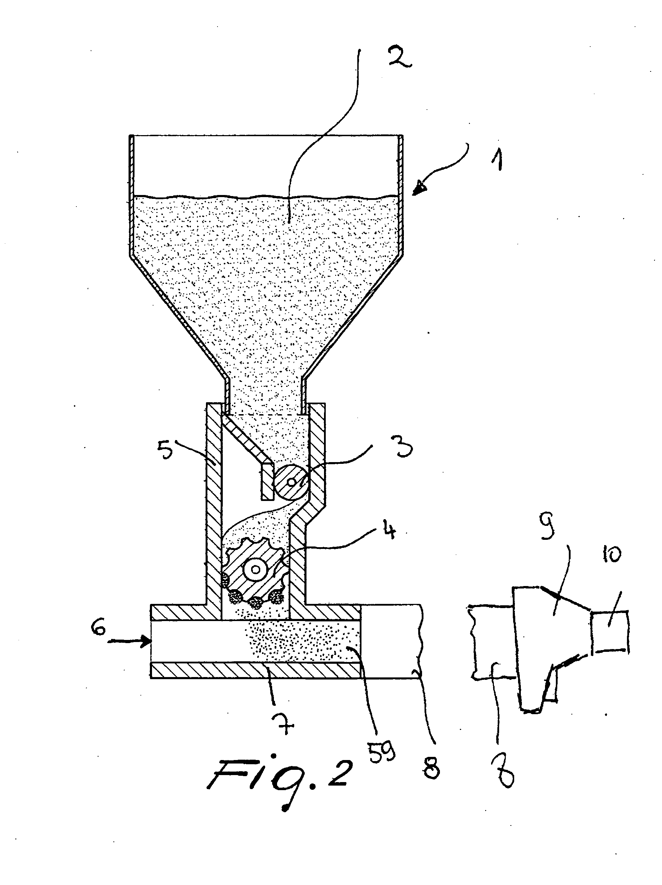

Unter Bezugnahme auf Fig. 2 wird eine schematische Darstellung einer ersten Vorrichtung zur Oberflächenbearbeitung bzw. Oberflächenbehandlung mittels Trockeneisgranulat erläutert, wobei gemeinsame Bestandteile zur Fig. 1 mit gleichen Bezugszeichen versehen werden. Die Vorrichtung umfasst einen im Wesentlichen trichterförmigen Vorratsbehälter 1 der mit Trockeneis-Pellets 2 (nachstehend zur Vereinfachung auch Pellets genannt) aufgefüllt ist. Die Pellets 2 haben, wie handelsüblich erhältlich, eine Größe von etwa 3 mm. Der Vorratsbehälter 2 mündet in ein radial ausgebildetes einstellbares Mahlwerk 3, welches die durch Schwerkraft aufliegenden 3 mm Pellets 2 in kleine Partikel zerkleinert, die in einem Bereich von etwa 1 mm bis etwa 100 µ liegen. Besonders bevorzugt ist ein Zerkleinerungsbereich der Pellets 2 in einer Größenordnung von etwa 200 µ bis etwa 400 µ und besonders bevorzugt sind Partikel, die etwa 300 µ groß sind.

Vorteilhaft ist das radiale Mahlwerk 3 ausgebildet, um scharfkantige zerkleinerte Partikel zu erzeugen. Die Scharfkantigkeit der zerkleinerten Partikel, die sich unterstützend auf die Reinigungsleistung auswirkt, kann durch eine angepasste Oberflächengeometrie der Brechwalze des Mahlwerks 3 erzielt werden.

Das radiale Mahlwerk 3 wird von einem nicht gezeigten Motor drehend angetrieben, wobei vorteilhaft die Menge der zerkleinerten Partikel pro Zeiteinheit durch die Regelung der Drehzahl des Motors bzw. der Drehzahl der Brechwalze des Mahlwerks 3 variierbar ist.

Die zerkleinerten Partikel aus dem Mahlwerk 3 treffen unter Einwirkung der Schwerkraft auf eine radial ausgebildete Transportwalze 4, die mit axial verlaufenden Einkerbungen 4' versehen ist. Vorzugsweise erstrecken sich die Einkerbungen 4' über die gesamte axiale Länge der Transportwalze 4. Darüber hinaus ist es erfindungsgemäß denkbar, senkrecht zu den axialen Einkerbungen 4' radial verlaufende (nicht gezeigte) Einkerbungen zu realisieren. Die Transportwalze 4 ist vorzugsweise ebenfalls motorisiert, um ihre Drehzahl zu regeln, damit die Menge der zerkleinerten Partikel pro Zeiteinheit variiert wird.

Wie in Fig. 2 gezeigt, werden die in die Einkerbungen aufgenommenen zerkleinerten Partikel zwischen der Transportwalze 4 und der senkrechten Wand 5 der Zufuhrkammer 57 gezwungen. Dementsprechend ist es erfindungsgemäß denkbar, die Form bzw. Scharfkantigkeit der zerkleinerten Partikel durch den Abstand zwischen der senkrechten Wand 5 und der Transportwalze 5 zu beeinflussen. Dies kann durch eine (nicht gezeigte) tulpen- bzw. trichterfömige Ausgestaltung der Wand 5 und durch eine relative senkrechte Verschiebung der Wand 5 und/oder der Transportwalze 4 bewerkstelligt werden, um somit den Abstand dazwischen zu variieren.

Durch die Rotationsbewegung der Transportwalze 4 fallen die zerkleinerten Partikel in einen um 90° versetzten Strömungskanal 7 der Vorrichtung, der in einer herkömmlichen Weise, wie z. B. in der DE 10 2004 045 770 B3 beschrieben, am Einlass 6 von einer Druckluftquelle 18 mit Druckluft beaufschlagt wird. Durch die Einwirkung von Druckluft gelangen die zerkleinerten Partikel in den Transportschlauch 8 und von dort aus in die Strahlpistole 9 bzw. die Lavaldüse 10 der Strahlpistole 9.

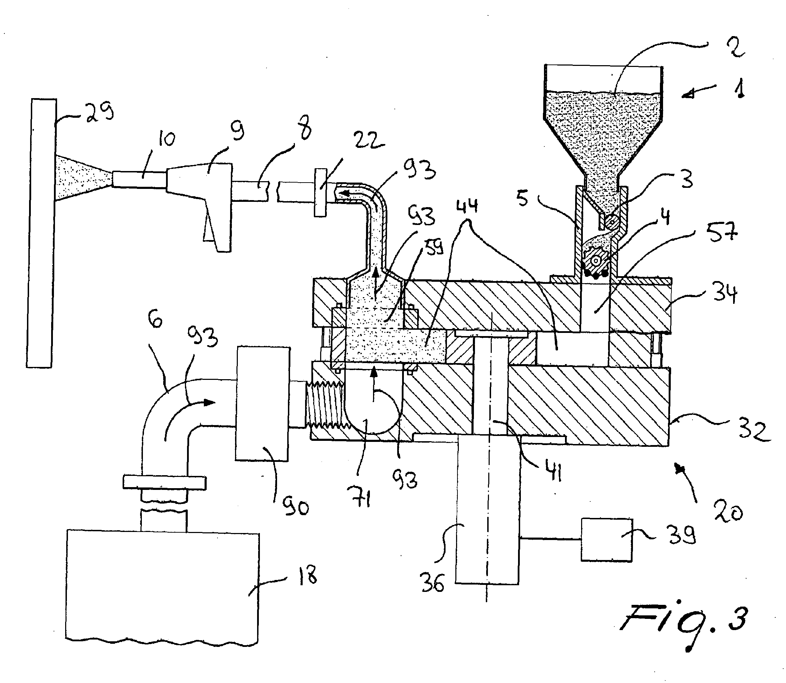

Unter Bezugnahme auf Fig. 3 wird eine schematische Darstellung einer zweiten Vorrichtung zur Oberflächenbearbeitung bzw. Oberflächenbehandlung mittels Trockeneisgranulat erläutert, wobei gemeinsame Bestandteile zu den Figuren 1 und 2 mit gleichen Bezugszeichen versehen werden.

Die zweite Vorrichtung setzt den Vorratsbehälter 1, das Mahlwerk 3 und die Transportwalze 4 der ersten Ausführungsform 1 ein, die in Verbindung mit einer Dosiereinrichtung 20 der DE 10 2004 045 770 B3 zum Einsatz kommt. Durch letztere Kombination kann besonders vorteilhaft eine zusätzliche Einstellung der Menge der zerkleinerten Partikel pro Zeiteinheit durch die Regelung der Drehzahl der Dosierscheibe 30 erzielt werden.

Sowohl in der ersten als auch in der zweiten Vorrichtung kann die Einstellung der Menge der zerkleinerten Partikel pro Zeiteinheit am Mahlwerk und/oder der Transportwalze und/oder der Dosiereinrichtung durch einen oder mehrere (nicht gezeigte) Regler an der Strahlpistole durchgeführt werden.

Durch die Zerkleinerung der 3 mm Pellets wird das Transportluftvolumen um 50% verringert, in Bezug auf das Transportluftvolumen, das erforderlich wäre, um herkömmliche Pellets in der gleichen Austrittsgeschwindigkeit, die typischerweise in einem Bereich von ca. 200 m/s - 300 m/s liegt, zu beschleunigen. Darüber hinaus erhöht sich durch die Zerkleinerung die effektive Menge an granuliertem Trockeneis, die um das 200-fache auf die zu behandelnde bzw. zu verarbeitende Fläche auftrifft und somit kann in Bezug auf herkömmliche Vorrichtungen die Flächenleistung drastisch erhöht werden.

Darüber hinaus sieht die erfindungsgemäße Verwendung eines Verfahrens nach Anspruch 1 vorteilhaft Schritte vor, um die Menge der zerkleinerten Partikel pro Zeiteinheit am Mahlwerk und/oder der Transportwalze und/oder der Dosiereinrichtung einzustellen. Dieses kann, wie vorstehend erwähnt, durch einen oder mehrere (nicht gezeigte) Regler an der Strahlpistole erfolgen.

Ein weiteres Merkmal ist die Bereitstellung von Schritten zur Einstellung der Form bzw. der Scharfkantigkeit der zerkleinerten Partikel, die wie vorstehend erwähnt an der Brechwalze des Mahlwerks und/oder an der Transportwalze erfolgen können.

Die Verwendung des Verfahrens nach Anspruch 1 betrifft die kosmetische Behandlung bzw. Reinigung von Haut.

## Patentansprüche

1. Verwendung eines Verfahrens für die kosmetische Behandlung bzw. Reinigung von Haut mittels Trockeneisgranulat, wobei das Verfahren mit einer Vorrichtung durchgeführt wird, folgendes umfassend: einen Vorratsbehälter (1) zur Aufnahme von Trockeneis-Pellets; einen Einlass (6) für die Zuführung von Druckluft; einen Auslass (22) zur Zuleitung eines Gemisches aus Druckluft und Trockeneisgranulat an eine Strahlpistole (9); und eine dazwischenliegende. Ausströmkämmer (59), wobei die Trockeneis-Pellets vor der Zuführung in die Ausströnikammer (59) zu einer Größe in einem Bereich zerkleinert werden, der von etwa 1 mm bis etwa 100 µ liegt.

2. Verwendung des Verfahrens nach Anspruch 1, wobei die Größe der zerkleinerten Trockeneis-Pellets in einem Bereich von etwa 200 µ bis etwa 400 µ liegt, und vorzugsweise etwa 300 µ beträgt.

3. Verwendung des Verfahrens nach Anspruch 1 oder 2, das weiterhin einen Schritt umfasst, um die Form der zerkleinerten Trockeneis-Pellets scharfkantig zu gestalten.

4. Verwendung des Verfahrens nach einem der Ansprüche 1 bis 3, das weiterhin einen Schritt umfasst, um die Menge der zerkleinerten Trockeneis-Pellets pro Zeiteinheit zu variieren.

## Claims

1. Use of a method for the cosmetic treatment or purification, respectively, of skin by means of dry-ice granulate, the method being performed with a device comprising: a reservoir (1) for receiving dry-ice pellets, an inlet (6) for feeding compressed air, an outlet (22) for feeding a mixture of compressed air and dry-ice granulate to a jet gun (9), and an outflow chamber (59) disposed therebetween, where the dry-ice pellets, before feeding into the outflow chamber (59), are crushed to a size within a range from approximately 1 mm to approximately 100 µ.

2. Use of the method according to claim 1, where the size of the crushed dry-ice pellets lies within a range from approximately 200 µ to approximately 400 µ and is preferably approximately 300 µ.

3. Use of the method according to claim 1 or 2, further comprising a step to form the crushed dry-ice pellets in a sharp-edged shape.

4. Use of the method according to one of the claims 1 through 3, further comprising a step to vary the amount of crushed dry-ice pellets per time unit.

## Revendications

1. Utilisation d'un procédé pour le traitement cosmétique et/ou le nettoyage de la peau, au moyen de granulat de glace sèche ou neige carbonique, le procédé étant mis en oeuvre avec un dispositif comprenant ce qui suit : un récipient de réserve (1), pour recevoir des granulés de neige carbonique ; une admission (6), pour l'amenée d'air comprimé : une évacuation (22), pour l'amenée à un pistolet de projection (9) d'un mélange formé d'air comprimé et de granulat de neige carbonique ; et une chambre de sortie d'écoulement (59) placée en position intermédiaire, les granulés de neige carbonique étant broyés avant l'amenée dans la chambre de sortie d'écoulement (59), pour être portés à une taille située dans une fourchette dimensionnelle allant d'à peu près 1 mm à à peu près 100 µm.

2. Utilisation du procédé selon la revendication 1, **caractérisé en ce que** la taille des granulés de neige carbonique broyés se situe dans une fourchette d'à peu près 200 µm à à peu près 400 µm et, de préférence, est d'à peu près 300 µm.

3. Utilisation du procédé selon la revendication 1 ou 2, comprenant en outre une étape pour conformer les granulés de neige carbonique broyés afin qu'ils aient des arêtes vives.

4. Utilisation du procédé selon l'une des revendications 1 à 3, comprenant en outre une étape pour faire varier la quantité, par unité de temps, de granulés de neige carbonique broyés.
